(19) **Europäisches Patentamt European Patent Office Office européen des brevets**

(11) **EP 4 524 187 A1**

(12) **EUROPEAN PATENT APPLICATION**
published in accordance with Art. 153(4) EPC

(43) Date of publication:
**19.03.2025 Bulletin 2025/12**

(21) Application number: **23936788.1**

(22) Date of filing: **23.11.2023**

(51) International Patent Classification (IPC):
*C08J 11/10* (2006.01)       *C08G 64/30* (2006.01)
*C07C 37/055* (2006.01)       *C07C 37/84* (2006.01)
*C07C 39/16* (2006.01)

(86) International application number:
**PCT/KR2023/018958**

(87) International publication number:
**WO 2025/028724 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.07.2023   KR 20230098843**

(71) Applicant: **LG CHEM, LTD.**
**Yeongdeungpo-gu**
**Seoul 07336 (KR)**

(72) Inventors:
• **LEE, Jeongbin**
  **Daejeon 34122 (KR)**

• **KIM, Jeongnam**
  **Daejeon 34122 (KR)**
• **KIM, Minju**
  **Daejeon 34122 (KR)**
• **HWANG, Jihwan**
  **Daejeon 34122 (KR)**
• **HONG, Mooho**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **METHOD OF PREPARING MONOMER COMPOSITION FOR SYNTHESIZING RECYCLED PLASTIC, AND MONOMER COMPOSITION FOR SYNTHESIZING RECYCLED PLASTIC, RECYCLED PLASTIC, AND MOLDED ARTICLE USING SAME**

(57) The present invention relates to a method for preparing a monomer composition for synthesizing recycled plastic, a monomer composition for synthesizing recycled plastic, a recycled plastic using the same, and a molded product using the same. The method comprising the steps of: subjecting a polycarbonate-based resin to a depolymerization reaction; adjusting the pH of the depolymerization reaction product having a pH of 13 or more to be 8 to 12; adjusting the pH of the depolymerization reaction product whose pH is adjusted to 8 to 12 to be less than 4; and adding a crystallization solvent to the depolymerization reaction product whose pH is adjusted to less than 4 and recovering the formed aromatic diol compound crystals.

# EP 4 524 187 A1

**Description**

**[TECHNICAL FIELD]**

<u>CROSS-REFERENCE TO RELATED APPLICATION(S)</u>

This application claims the benefit of Korean Patent Application No. 10-2023-0098843 filed on July 28, 2023 with the Korean Intellectual Property Office, the disclosure of which is incorporated herein by reference in its entity.

The present invention relates to a method for preparing a monomer composition for synthesizing recycled plastic that improves the efficiency of a recycling process while enabling an aromatic diol compound to have good yield and optical properties, with the aromatic diol compound being recovered through recycling by chemical decomposition of a polycarbonate-based resin, and to a monomer composition for synthesizing recycled plastic, a recycled plastic and a molded product using the same.

**[BACKGROUND]**

Polycarbonate is a thermoplastic polymer and is a plastic having excellent characteristics such as excellent transparency, superior ductility, and relatively low production costs.

Although polycarbonate is widely used for various purposes, environmental and health concerns during waste treatment have been continuously raised.

Currently, a physical recycling method is carried out, but in this case, a problem accompanying the deterioration of the quality occurs, and thus, research on the chemical recycling of polycarbonate is underway.

Chemical decomposition of polycarbonate refers to obtaining an aromatic diol compound as a monomer (e.g., bisphenol A (BPA)) through decomposition of polycarbonate, and then utilizing it again in polymerization to obtain a high-purity polycarbonate.

For such a chemical decomposition, thermal decomposition, hydrolysis, and alcohol decomposition are typically known. Among these, the most common method is alcohol decomposition using a base catalyst, but in the case of methanol decomposition, there is a problem that methanol is used which is harmful to the human body, and in the case of ethanol, there is a problem that high temperature and high pressure conditions are required and the yield is not high.

In addition, although an alcohol decomposition method using an organic catalyst is known, it is disadvantageous in terms of economics.

**[DETAILED DESCRIPTION OF THE INVENTION]**

**[Technical Problem]**

It is an object of the present invention to provide a method for preparing a monomer composition for synthesizing recycled plastic that improves the efficiency of a recycling process while enabling an aromatic diol compound to have good yield and optical properties, with the aromatic diol compound being recovered through recycling by chemical decomposition of a polycarbonate-based resin.

It is another object of the present invention to provide a monomer composition for synthesizing recycled plastic, a recycled plastic and a molded product using the method for preparing a monomer composition for synthesizing recycled plastic.

**[Technical Solution]**

In order to achieve the above object, provided herein is a method for preparing a monomer composition for synthesizing recycled plastic, the method comprising the steps of: subjecting a polycarbonate-based resin to a depolymerization reaction; adjusting the pH of the depolymerization reaction product having a pH of 13 or more to be 8 to 12; adjusting the pH of the depolymerization reaction product whose pH is adjusted to 8 to 12 to be less than 4; and adding a crystallization solvent to the depolymerization reaction product whose pH is adjusted to less than 4 and recovering the formed aromatic diol compound crystals.

Also provided herein is a monomer composition for synthesizing recycled plastic, comprising the aromatic diol compound obtained by the method for preparing a monomer composition for synthesizing recycled plastic.

Further provided herein is a recycled plastic comprising a reaction product of the monomer composition for synthesizing recycled plastic and a comonomer.

Further provided herein is a molded product comprising the recycled plastic.

Below, a method for preparing a monomer composition for synthesizing recycled plastic, and a monomer composition for synthesizing recycled plastic, a recycled plastic and a molded product using the same according to specific embodiments of the present invention will be described in more detail.

Unless explicitly stated herein, the technical terms used herein are for the purpose of describing specific embodiments only and is not intended to limit the scope of the invention.

The singular forms "a," "an" and "the" used herein are intended to include plural forms, unless the context clearly indicates otherwise.

The 'pH' as used herein means a hydrogen ion concentration (pH), which is a numerical value indicating the acidity and alkalinity of a material. The pH can be determined from a value expressed by taking the reciprocal of the logarithmic dissociation concentration of hydrogen ions, and is used as a measure of the strength of acids and bases of a material.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present invention.

## 1. Method for preparing a monomer composition for synthesizing recycled plastic

According to one embodiment of the present invention, there can be provided a method for preparing a monomer composition for synthesizing recycled plastic, the method comprising the steps of: subjecting a polycarbonate-based resin to a depolymerization reaction; adjusting the pH of the depolymerization reaction product having a pH of 13 or more to be 8 to 12; adjusting the pH of the depolymerization reaction product whose pH is adjusted to 8 to 12 to be less than 4; and adding a crystallization solvent to the depolymerization reaction product whose pH is adjusted to less than 4 and recovering the formed aromatic diol compound crystals.

The present inventors have found through experiments that in the same way as the method for preparing a monomer composition for synthesizing recycled plastic according to the one embodiment, the pH of the depolymerized polycarbonate-based resin is adjusted stepwise in two steps in the process of recycling polycarbonate-based resin through chemical decomposition, and the organic solvent can be separated and removed to a sufficient level in the neutralization step, whereby high-purity aromatic diol compound crystals can be immediately recovered in high yield through recrystallization without removing the organic solvent through a separate distillation process, and completed the present invention.

In particular, conventionally, as a strong acid aqueous solution is used in the neutralization step to lower the pH at one time to a neutral or acidic level, organic solvents such as ethanol or diethyl carbonate are not sufficiently separated or removed and remained, and thus, a distillation step is required to remove such organic solvents. Further, due to the solubility between diethyl carbonate and ethanol, there is a limitation in that it is difficult to recrystallize and precipitate bisphenol A, which is an aromatic diol compound.

On the other hand, in the present invention, a multi-step neutralization process progresses in which the pH is lowered once to 8 to 12 in the neutralization step, and then the pH is sequentially lowered to less than 4. Therefore, by adding an acidic aqueous solution during the process of lowering the pH to 8 to 12 and then removing the separated aqueous layer, impurities that dissolve in water such as salts can be easily removed, and the color characteristics of the monomer composition for recycled plastic synthesis can be improved. Furthermore, while increasing in the solubility of ethanol in water, it is possible to effectively realize the effect of removing ethanol remaining in the organic layer without a distillation step. In addition, the effect of sufficiently progressing recrystallization and precipitation of bisphenol A, which is an aromatic diol compound, is realized even with a small amount of recrystallization solvent through the process of separating the water layer and the organic solvent layer in multiple steps.

Specifically, the method for preparing a monomer composition for synthesizing recycled plastic according to one embodiment may include a step of subjecting a polycarbonate-based resin to a depolymerization reaction.

The polycarbonate-based resin is meant to include both a homopolymer and a copolymer containing a polycarbonate repeating unit, and collectively refers to a reaction product obtained through a polymerization reaction or a copolymerization reaction of a monomer containing an aromatic diol compound and a carbonate precursor. When it contains one type of carbonate repeating unit obtained by using only one type of aromatic diol compound and one type of carbonate precursor, a homopolymer can be synthesized. In addition, when one type of aromatic diol compound and two or more types of carbonate precursors are used as the monomer, or two or more types of aromatic diol compounds and one type of carbonate precursor are used, or one or more types of other diols is used in addition to the one type of aromatic diol compound and the one type of carbonate precursor to contain two or more types of carbonates, a copolymer can be synthesized. The homopolymer or copolymer can include all of low-molecular compounds, oligomers, and polymers depending on the molecular weight range.

The polycarbonate-based resin can be applied regardless of various forms and types, such as a novel polycarbonate-based resin produced through synthesis, a recycled polycarbonate-based resin produced through a recycling process, or polycarbonate-based resin waste.

However, if necessary, before subjecting the polycarbonate-based resin to a depolymerization reaction, a pretreatment step of the polycarbonate-based resin is performed, thereby capable of increasing the efficiency of the process of recovering the aromatic diol compound and the carbonate precursor from the polycarbonate-based resin. Examples of the pretreatment step may include washing, drying, grinding, glycol decomposition, and the like. The specific method of each pretreatment step is not limited, and various methods widely used in the process of recovering the aromatic diol compound and the carbonate precursor by the depolymerization of the polycarbonate-based resin can be applied without limitation.

During the depolymerization reaction of the polycarbonate-based resin, the depolymerization reaction may be carried out under acidic, neutral or basic conditions, and particularly, the depolymerization reaction may be carried out under basic (alkali) conditions. The type of the base is not particularly limited, and examples thereof include sodium hydroxide (NaOH) or potassium hydroxide (KOH). The base is a base catalyst acting as a catalyst, and has the economic advantages over organic catalysts, which are mainly used under mild conditions. More specifically, during the depolymerization reaction of the polycarbonate-based resin, the depolymerization reaction may proceed at a pH of 13 or more, or in the range of 13 to 14.

During the depolymerization reaction of the polycarbonate-based resin, the depolymerization reaction may be carried out by reacting a base in an amount of 0.5 mole or less, or 0.4 mole or less, or 0.3 mole or less, or 0.1 mole or more, or 0.2 mole or more, or 0.1 mole to 0.5 mole, or 0.1 mole to 0.4 mole, or 0.1 mole to 0.3 mole, or 0.2 mole to 0.5 mole, or 0.2 mole to 0.4 mole, or 0.2 mole to 0.3 mole relative to 1 mole of the polycarbonate-based resin. When the polycarbonate-based resin is reacted with a base in an amount of more than 0.5 mole relative to 1 mole of the polycarbonate-based resin during depolymerization of the polycarbonate-based resin, there is a limitation in that impurities increase due to the effect of increasing the amount of alkali salt generated, so that the purity of the target recovery material is reduced, and the economic efficiency of the catalytic reaction is reduced.

Further, the depolymerization reaction of the polycarbonate-based resin can be performed in the presence of a solvent including ethanol. The present invention can stably obtain bisphenol A, which is a high-purity monomer, by decomposing a polycarbonate-based resin with a solvent including ethanol, and has the advantage that diethyl carbonate having a high added value can be further obtained as a reaction byproduct.

The content of the ethanol may be 1 mole to 5 moles, or 1 mole to 4 moles, or 1 mole to 3.75 moles relative to 1 mole of the polycarbonate-based resin. Since the ethanol has good solubility in bisphenol A, ethanol within the above range should be essentially contained. When the content of the ethanol is excessively reduced to less than 1 mole relative to 1 mole of the polycarbonate-based resin, it is difficult to sufficiently progress the alcohol decomposition of the polycarbonate-based resin. On the other hand, when the content of ethanol is excessively increased relative to 1 mole of the polycarbonate-based resin, it is difficult to remove ethanol sufficiently in the neutralization step, which will be described later, and the economic efficiency of the process may be reduced as a separate distillation step is involved.

The solvent in which the depolymerization reaction of the polycarbonate-based resin proceeds may further include, in addition to ethanol, at least one organic solvent selected from the group consisting of tetrahydrofuran, toluene, methylene chloride, chloroform, dimethyl carbonate, ethylmethyl carbonate, diethyl carbonate, and dipropyl carbonate.

The organic solvent may include tetrahydrofuran, toluene, methylene chloride, chloroform, dimethyl carbonate, ethylmethyl carbonate, diethyl carbonate, dipropyl carbonate, or a mixture of two or more thereof.

More preferably, methylene chloride can be used as the organic solvent. When methylene chloride is used as an organic solvent to be mixed with the ethanol, there is an advantage that the dissolution properties in polycarbonate can be improved and the reactivity can be enhanced.

The content of the organic solvent may be 10 moles to 20 moles, or 10 moles to 15 moles relative to 1 mole of the polycarbonate-based resin. In addition, the content of the organic solvent may be 2.2 moles to 5 moles relative to 1 mole of ethanol. By mixing the polycarbonate-based resin, ethanol, and an organic solvent within the above range, there is an advantage that the depolymerization reaction of the polymer can proceed at a desired level.

Meanwhile, the temperature at which the depolymerization reaction of the polycarbonate-based resin proceeds is not particularly limited, but for example, the reaction may proceed at a temperature of 20°C to 100°C, or 70°C to 90°C. In addition, the depolymerization reaction of the polycarbonate-based resin may proceed for 1 hour to 30 hours.

Specifically, the conditions are mild process conditions relative to the conventional pressurizing/high temperature process, and by performing stirring under the above conditions, the process can be performed in a mild process as compared to the pressurizing/high temperature process.

That is, according to the present invention, as the type and mixing amount of the mixed solvent and the type and content of the base catalyst is adjusted without using an organic catalyst, there is the advantage that a high-purity aromatic diol compound (e.g., bisphenol A) can be obtained under mild conditions without using a pressurizing/high temperature process, and an ethanol solvent is used and thus, diethyl carbonate can be obtained as by-products.

Meanwhile, during the depolymerization reaction of the polycarbonate-based resin, an antioxidant can be added to the reaction solution. As the antioxidant is added, the aromatic diol compound recovered through recycling by chemical decomposition of the polycarbonate-based resin can satisfy a color coordinate b* value at a color level comparable to reagents commercially sold or used for a PC polymerization.

Specific examples of the antioxidant are not particularly limited, and various antioxidants that have been widely used in the prior art can be applied without limitation. However, one example thereof include sodium hyposulfite, sodium sulfite, erythorbic acid, dibutylhydroxytoluene, butylhydroxyanisole, $\alpha$-tocopherol, tocopherol acetate, L-ascorbic acid and salts thereof, L-ascorbic acid palmitate, L-ascorbic acid stearate, triamyl gallate, propyl gallate or ethylenediamine tetraacetic acid disodium salt(EDTA), sodium pyrophosphate, sodium metaphosphate, or a mixture of two or more thereof.

The specific addition amount of the antioxidant is also not particularly limited, but as an example, the antioxidant can be added at a level that does not affect the physical properties of the monomer composition for synthesizing recycled plastic within the range of 0.1% by weight to 5% by weight, or 0.1% by weight to 1% by weight based on the total weight of the reaction solution.

Meanwhile, the method for preparing a monomer composition for synthesizing recycled plastic according to one embodiment may comprise a step of adjusting the pH of the depolymerization reaction product having a pH of 13 or more, or 13 to 14, to be 8 to 12, or 8 to 11, or 8 to 10, or 8 to 9.

Specifically, the step of adjusting the pH of the depolymerization reaction product to be 8 to 12 may comprise a step of adding an acidic aqueous solution so that the pH of the depolymerization reaction product is 8 to 12. Through the step of adjusting the pH of the depolymerization reaction product to be 8 to 12, water-soluble salt impurities can be effectively removed and color characteristics can be improved. In addition, the solubility of unreacted ethanol remaining in the depolymerization reaction product in water can be increased, and the amount of residual ethanol can be minimized.

Specifically, a layer divided into an organic solvent layer and a water layer may be formed in the step of adding an acidic aqueous solution so that the pH of the depolymerization reaction product is 8 to 12. More specifically, the layer divided into the organic solvent layer and the water layer may form a layer divided into a water layer containing impurities, and an organic solvent layer containing an aromatic diol compound.

Since the aromatic diol compound has hydrophobicity, it may be included in an organic solvent layer among water layer and an organic solvent layer, and various water-soluble impurities can be included in the water layer. The impurity is a material having hydrophilicity, and examples thereof may include a salt compound, an ionic compound, an acid compound, and the like. Further, the water layer containing impurities may further include unreacted ethanol.

The acidic aqueous solution is a mixed solution of acid and water, wherein the acid may be a strong acid, such as hydrochloric acid (HCl). As the depolymerization reaction proceeds under strong basic conditions with a pH of 13 or more, the resulting aromatic diol compound exists in the form of a salt through reaction with a base, and thus has hydrophilicity. However, by adding an acidic aqueous solution, the salt of the aromatic diol compound contained in the depolymerization reaction product can be converted into the aromatic diol compound, thereby inducing to have hydrophobicity.

Meanwhile, after the step of adjusting the pH of the depolymerization reaction product having a pH of 13 or more to be 8 to 12, the method may further comprise a step of removing a water layer among the layers divided into an organic solvent layer and a water layer. Since various water-soluble impurities are separated in the water layer among the layers divided into the water layer and the organic solvent layer by adding the acidic aqueous solution, impurities can be easily removed from an aromatic diol compound as the main product only by a simple process of removing the water layer.

Specific conditions for removing the water layer from the organic layer are not particularly limited. As for the specific removal devices and methods, various well-known purification techniques can be applied without limitation. However, as an example, a drain device can be used.

Meanwhile, the method for preparing the monomer composition for synthesizing recycled plastic according to the one embodiment may comprise a step of adjusting the pH of the depolymerization reaction product whose pH is adjusted to 8 to 12 to be less than 4, or 1 to 3, or 1 to 2.

Specifically, the step of adjusting the pH of the depolymerization reaction product whose pH is adjusted to 8 to 12 to be less than 4 may comprise a step of adding an acidic aqueous solution so that the pH of the depolymerization reaction product is less than 4. Through the step of adding an acidic aqueous solution so that the pH of the depolymerization reaction product is less than 4, the salt of the aromatic diol compound remaining in the depolymerization reaction product whose pH is adjusted to 8 to 12 can be converted into an aromatic diol compound, thereby increasing the yield of the aromatic diol compound.

Specifically, a layer divided into an organic solvent layer and a water layer may be formed in the step of adding an acidic aqueous solution so that the pH of the depolymerization reaction product is less than 4. More specifically, the layer divided into the organic solvent layer and the water layer may form a layer divided into a water layer containing impurities, and an organic solvent layer containing an aromatic diol compound.

Since the aromatic diol compound has hydrophobicity, it may be included in an organic solvent layer among water and an organic solvent, and various water-soluble impurities can be included in the water layer. The impurity is a material having hydrophilicity, and examples thereof may include a salt compound, an ionic compound, an acid compound, and the like.

The acidic aqueous solution is a mixed solution of acid and water, wherein the acid may be a strong acid, such as hydrochloric acid (HCl). By adding an acidic aqueous solution to the depolymerization reaction product whose pH is adjusted to 8 to 12, the salt of the aromatic diol compound remaining in the depolymerization reaction product can be converted into the aromatic diol compound, thereby inducing to have hydrophobicity.

Meanwhile, after the step of adjusting the pH of the depolymerization reaction product whose pH was adjusted to 8 to 12 to be less than 4, the method may further comprise a step of removing a water layer among the layers divided into an organic solvent layer and a water layer. Since various water-soluble impurities are separated in the water layer among the layers divided into the water layer and the organic solvent layer by adding the acidic aqueous solution, impurities can be easily removed from an aromatic diol compound as the main product only by a simple process of removing the water layer.

Specific conditions for removing the water layer from the organic layer are not particularly limited. As for the specific removal devices and methods, various well-known purification techniques can be applied without limitation. However, as an example, a drain device can be used.

Meanwhile, the pH difference value according to the following Equation 1 may be 4 or more, or 4 to 10, or 5 to 8, or 6 to 7.

$$\text{pH difference value} = (\text{pH of the depolymerization reaction product whose pH --> is adjusted to 8 to 12}) - (\text{pH of the depolymerization reaction product whose pH is adjusted to less than 4}). \qquad \text{[Equation 1]}$$

As the pH difference value according to Equation 1 satisfies 4 or more, or 4 to 10, or 5 to 8, or 6 to 7, it is possible to further maximize the effect of removing salt impurities and residual ethanol caused by adjusting the pH to 8 to 12, and the effect of improving the yield of the aromatic diol compound caused by adjusting the pH to less than 4.

Further, the pH difference value according to the following Equation 2 may be 1 to 6, or 2 to 6, or 3 to 6, or 4 to 6, or 5 to 6.

$$\text{pH difference value} = (\text{pH of the depolymerization reaction product}) - (\text{pH of the depolymerization reaction product whose pH is adjusted to 8 to 12}). \qquad \text{[Equation 2]}$$

As the pH difference value according to Equation 2 satisfies 1 to 6, or 2 to 6, or 3 to 6, or 4 to 6, or 5 to 6, it is possible to further maximize the effect of removing salt impurities and residual ethanol caused by adjusting the pH to 8 to 12.

Specifically, the ratio of residual ethanol contained in the depolymerization reaction product whose pH is adjusted to less than 4 according to the following Equation 3 may be less than 1%, or 0.1% to 0.9%, or 0.1% to 0.8%, or 0.1% to 0.7%, or 0.1% to 0.6%, or 0.1% to 0.5%.

$$\text{Residual ethanol ratio (\%)} = \{(\text{ethanol peak area in } {}^{1}\text{H NMR}) / (\text{ethanol peak area in } {}^{1}\text{H NMR} + \text{aromatic diol compound peak area in } {}^{1}\text{H NMR})\} \times 100. \qquad \text{[Equation 3]}$$

Specifically, in Equation 3, the ethanol peak in $^{1}$H NMR is targeted at the peak at 3.63 ppm (quartet, 2H), and the aromatic diol compound (bisphenol A (BPA)) peak in $^{1}$H NMR is targeted at the peak at 6.67 ppm (4H).

As the residual amount of ethanol is reduced in this way, ethanol is sufficiently removed and the aromatic diol compound can be precipitated in high purity and yield without the need for a separate distillation process, thereby improving the economic efficiency of the process.

On the other hand, in case where the residual amount of ethanol contained in the depolymerization reaction product whose pH is adjusted to less than 4 according to Equation 3 increases to 1% or more, even if a large amount of crystallization solvent is added to the extent that precipitation of the aromatic diol compound can be advanced only through a distillation process to remove ethanol, the yield of the aromatic diol compound obtained through precipitation is reduced, which limits the efficiency of the process.

Meanwhile, the method for preparing a monomer composition for synthesizing recycled plastic according to the one embodiment may comprise a step of adding a crystallization solvent to the depolymerization reaction product whose pH is adjusted to less than 4 and recovering the formed aromatic diol compound crystals.

As the crystallization solvent is added to precipitate the aromatic diol compound in this way, the aromatic diol compound, which is the main synthesis target material in the present invention, can be secured with high purity and high yield.

The step of adding a crystallization solvent to the depolymerization reaction product whose pH is adjusted to less than 4 and recovering the formed aromatic diol compound crystals may comprise a step of adding water to the depolymerization reaction product whose pH is adjusted to less than 4 to precipitate aromatic diol compound crystals, wherein the water is added in an amount of 100 mol or less, or 50 moles to 100 moles, or 50 moles to 90 moles, or 50 moles to 80 moles, or 50 moles to 70 moles relative to 1 mole of the polycarbonate resin.

When the amount of water added during extraction of the aromatic diol compound is excessively reduced, the aromatic diol compound may not be sufficiently extracted and thus, the yield may decrease. On the other hand, when the amount of water added during extraction of the aromatic diol compound is excessively increased, excessive water may be required, which may reduce the efficiency of the extraction process.

Meanwhile, the method for preparing a monomer composition for synthesizing recycled plastic according to one embodiment may further comprise a step of purifying the aromatic diol compound, after the step of adding a crystallization solvent to the depolymerization reaction product whose pH is adjusted to less than 4 and recovering the formed aromatic diol compound crystals.

Specifically, the step of purifying the aromatic diol compound may comprise a step of washing the aromatic diol compound. Further, the step of purifying the aromatic diol compound may comprise a step of dissolving the aromatic diol compound and a step of subjecting it to adsorption purification.

The order of the washing step; and the adsorption purification step is not particularly limited, and it is irrelevant to proceed in any order, but as an example, it can proceed in the order of the washing step; and the adsorption purification step. The washing step; and the adsorption purification step can proceed repeatedly at least once or more. As for the addition of specific washing, adsorption devices and methods, various well-known purification techniques can be applied without limitation.

In the washing step of the aromatic diol compound, since various impurities remain during the recovery process of obtaining the aromatic diol compound, washing can proceed in order to sufficiently remove these impurities and secure a high-purity aromatic diol compound.

Specifically, the washing step may comprise a step of washing with a solvent at a temperature of 10°C to 30°C, or 20°C to 30°C. The temperature condition means the temperature inside a washing container at which washing with a solvent is performed. In order to maintain a high temperature deviating from a room temperature, various heating devices can be applied without limitation.

The solvent used in the washing step may include one of water, alcohol, and an organic solvent. As the organic solvent, tetrahydrofuran, toluene, methylene chloride, chloroform, dimethyl carbonate, ethylmethyl carbonate, diethyl carbonate, dipropyl carbonate, or a mixture of two or more thereof can be used.

The solvent used in the washing step can be used in a weight ratio of 1 part by weight or more and 30 parts by weight or less, or 1 part by weight or more and 10 parts by weight or less, based on 1 part by weight of the polycarbonate-based resin used in the depolymerization reaction.

More specifically, the solvent in the step of washing with a solvent at a temperature of 10°C or more and 30°C or less may be an organic solvent. Methylene chloride can be preferably used as the organic solvent.

Meanwhile, the dissolution step and the adsorption purification step of the aromatic diol compound may include a dissolution step of the aromatic diol compound and an adsorption purification step of the aromatic diol compound.

The dissolution step of the aromatic diol compound may include a step of adding a solvent to the aromatic diol compound. Examples of the solvent include ethanol, and the ethanol may be added in a ratio of 1 mole to 20 moles, or 1 mole to 10 moles, or 5 moles to 10 moles, based on 1 mole of the polycarbonate-based resin. Through the step of adding a solvent to the aromatic diol compound, the aromatic diol compound crystals may be redissolved in the solvent.

The adsorption purification step of the aromatic diol compound may include a step of adding an adsorbent to the aromatic diol compound to perform an absorption purification and then removing the adsorbent. In the step of adding an adsorbent to the aromatic diol compound to perform an absorption purification and then removing the adsorbent, an adsorbent can be brought into contact with the aromatic diol compound.

Examples of the adsorbent that can be used include activated carbon, charcoal, or a mixture thereof. The activated carbon is a black carbon material having micropores produced by subjecting a raw material to a carbonization process at about 500°C and an activated carbon process at about 900°C, and examples thereof are not particularly limited, but for example, various activated carbons such as plant-based, coal-based, petroleum-based, waste-based activated carbons can be applied without limitation depending on the type of raw material.

In more specific examples, the plant-based activated carbon may include coconut activated carbon, wood activated carbon, and sawdust activated carbon. Further, the coal-based activated carbon may include lignite activated carbon, bituminous coal activated carbon, and anthracite activated carbon. Further, the petroleum-based activated carbon may include petroleum coke activated carbon and oil carbon activated carbon. Further, the waste activated carbon may include synthetic resin activated carbon and pulp activated carbon.

The adsorbent may include at least one activated carbon selected from the group consisting of plant-based activated carbon, coal-based activated carbon, petroleum-based activated carbon, and waste-based activated carbon. That is, the adsorbent may include plant-based activated carbon, coal-based activated carbon, petroleum-based activated carbon, waste-based activated carbon, or a mixture of two or more thereof.

More specifically, the adsorbent may include at least one activated carbon selected from the group consisting of coconut activated carbon, lignite activated carbon, anthracite activated carbon, and bituminous coal activated carbon. That is, the adsorbent may include coconut activated carbon, lignite activated carbon, anthracite activated carbon, bituminous coal activated carbon, or a mixture of two or more thereof.

The adsorption purification conditions by the adsorbent are not particularly limited, and various well-known adsorption purification conditions can be used without limitation. However, in one example, the addition amount of the adsorbent may be 10% by weight to 50% by weight relative to the polycarbonate-based resin, the adsorption time may be 1 hour to 5 hours, and the adsorption method may be a stirring adsorption or an adsorption tower for Lab.

Meanwhile, after the dissolution step and the adsorption purification step of the aromatic diol compound, a recrystallization step of the aromatic diol compound may be further included. In the recrystallization step of the aromatic diol compound, a high-purity aromatic diol compound can be secured by sufficiently removing various impurities contained in the aromatic diol compound.

Specifically, the recrystallization step may include a step of adding water to the solution in which the aromatic diol compound is dissolved and recrystallizing it. Through the step of adding water to the solution in which the aromatic diol compound is dissolved and recrystallizing it, the solubility of the aromatic diol compound, or its salt contained in the solution in which the aromatic diol compound is dissolved is increased, and thus, crystals, or impurities interposed between crystals can be dissolved with a solvent to the maximum, and further, since the dissolved aromatic diol compound has poor solubility relative to impurities, it can be easily precipitated into aromatic diol compound crystals through the difference in solubility when the temperature is lowered subsequently.

More specifically, in the step of adding water to the solution in which the aromatic diol compound is dissolved and recrystallizing it, 20 moles to 40 moles, or 25 moles to 35 moles of water can be used with respect to 1 mole of the polycarbonate-based resin. When the water is used in an excessively small amount, the temperature for dissolving the aromatic diol compound contained in the solution in which the aromatic diol compound is dissolved becomes too high, which thus deteriorates in the process efficiency, and it is difficult to remove impurities through recrystallization. On the other hand, when water is used in an excessively large amount, the solubility of the aromatic diol compound contained in the solution in which the aromatic diol compound is dissolved becomes too high, and thus, the yield of the aromatic diol compound recovered after recrystallization is reduced, and the process efficiency can be reduced due to the use of large amounts of solvent.

If necessary, after proceeding the recrystallization step of the aromatic diol compound is dissolved, a step of removing residual impurities through filtration or adsorption may be further performed.

In addition, if necessary, after the recrystallization step, the method may further include a drying step. The remaining solvent can be removed by the drying, and the specific drying conditions are not particularly limited, but for example, the drying can be performed at a temperature of 10°C to 100°C, or 10°C to 50°C. As for the specific drying equipment and method used in the drying, various well-known drying techniques can be applied without limitation.

## 2. Monomer Composition for Synthesizing Recycled Plastic

According to another embodiment of the present invention, there can be provided a monomer composition for synthesizing recycled plastic, comprising the aromatic diol compound obtained by the method for preparing a monomer composition for synthesizing recycled plastic according to the one embodiment.

The monomer composition for synthesizing recycled plastic according to another embodiment may be obtained by the method for preparing a monomer composition for synthesizing recycled plastic according to the one embodiment. The details of the method for preparing the monomer composition for synthesizing recycled plastic according to the other embodiment include all the contents described above in the one embodiment.

That is, the monomer composition for synthesizing recycled plastic according to the other embodiment corresponds to the result obtained through various processes of filtration, purification, washing, and drying in order to secure only the aromatic diol compound, which is the main recovery target material, with high purity after the depolymerization reaction of the polycarbonate-based resin.

Further, specific examples of the aromatic diol compound include bis(4-hydroxyphenyl)methane, bis(4-hydroxyphenyl)ether, bis(4-hydroxyphenyl)sulfone, bis(4-hydroxyphenyl)sulfoxide, bis(4-hydroxyphenyl)sulfide, bis(4-hydroxyphenyl)ketone, 1,1-bis(4-hydroxyphenyl)ethane, 2,2-bis(4-hydroxyphenyl)propane (bisphenol A), 2,2-bis(4-hydroxyphenyl)butane, 1,1-bis(4-hydroxyphenyl)cyclohexane (bisphenol Z), 2,2-bis(4-hydroxy-3,5-dibromophenyl)propane, 2,2-bis(4-hydroxy-3,5-dichlorophenyl)propane, 2,2-bis(4-hydroxy-3-bromophenyl)propane, 2,2-bis(4-hydroxy-3-chlorophenyl)propane, 2,2-bis(4-hydroxy-3-methylphenyl)propane, 2,2-bis(4-hydroxy-3,5-dimethylphenyl)propane, 1,1-bis(4-hydroxyphenyl)-1-phenylethane, or a mixture of two or more thereof, and the like. Preferably, the aromatic diol compound of the monomer composition for synthesizing recycled plastics according to the one embodiment may be 2,2-bis(4-hydroxyphenyl)propane (bisphenol A).

The aromatic diol compound is characterized by being obtained by the method for preparing the monomer composition for synthesizing the recycled plastic according to the one embodiment. That is, the aromatic diol compound means being recovered from the polycarbonate-based resin used for recovering the monomer composition for synthesizing recycled plastic. Therefore, apart from the recovery from the polycarbonate-based resin in order to prepare the monomer composition for synthesizing recycled plastics according to the one embodiment, the case where a novel aromatic diol compound is added from the outside is not included in the category of aromatic diol compound of the present invention.

Specifically, "being recovered from the polycarbonate-based resin" means being obtained through a depolymerization reaction of the polycarbonate-based resin. The depolymerization reaction can be performed under acidic, neutral or basic conditions, and particularly, the depolymerization reaction can proceed under basic (alkaline) conditions. Particularly, the depolymerization reaction can be preferably performed in the presence of an ethanol solvent, as will be described later.

**3. Recycled Plastic**

According to another embodiment of the invention, there can be provided a recycled plastic comprising a reaction product of the monomer composition for synthesizing recycled plastic according to the other embodiment and a comonomer.

The details of the monomer composition for synthesizing recycled plastic according to the other embodiment include all the contents described above in the other embodiment.

Examples corresponding to the recycled plastic are not particularly limited, and various plastics synthesized from aromatic diol compounds such as bisphenol A and carbonate precursors such as dimethyl carbonate, diethyl carbonate, or ethylmethyl carbonate as the monomer can be applied without limitation, and a more specific example may be a polycarbonate-based resin.

The polycarbonate-based resin is meant to include both a homopolymer and a copolymer containing a polycarbonate repeating unit, and collectively refers to a reaction product obtained through a polymerization reaction or a copolymerization reaction of a monomer containing an aromatic diol compound and a carbonate precursor. When it contains one type of carbonate repeating unit obtained by using only one type of aromatic diol compound and one type of carbonate precursor, a homopolymer can be synthesized. In addition, when one type of aromatic diol compound and two or more types of carbonate precursors are used as the monomer, or two or more types of aromatic diol compounds and one type of carbonate precursor are used, or one or more types of other diols is used in addition to the one type of aromatic diol compound and the one type of carbonate precursor to contain two or more types of carbonates, a copolymer can be synthesized. The homopolymer or copolymer can include all of low-molecular compounds, oligomers, and polymers depending on the molecular weight range.

More specifically, in the recycled plastic containing the reaction product of the monomer composition for synthesizing the recycled plastic and the comonomer according to the one embodiment, a carbonate precursor can be used as the comonomer. Specific examples of the carbonate precursor include phosgene, triphosgene, diphosgene, bromophosgene, dimethyl carbonate, diethyl carbonate, dibutyl carbonate, dicyclohexyl carbonate, diphenyl carbonate, ditolyl carbonate, bis(chlorophenyl)carbonate, m-cresyl carbonate, dinaphthyl carbonate, bis(diphenyl) carbonate or bishaloformate.

Examples of the reaction process of a monomer composition for synthesizing recycled plastic and a comonomer that synthesize the polycarbonate-based resin are not particularly limited, and various well-known methods for preparing the polycarbonate can be applied without limitation.

However, in one example of the polycarbonate preparation method, a polycarbonate preparation method including the step of polymerizing a composition containing a monomer composition for synthesizing recycled plastic and a comonomer can be used. At this time, the polymerization can be carried out by interfacial polymerization, and during interfacial polymerization, polymerization reaction is possible at normal pressure and low temperature, and it is easy to adjust the molecular weight.

The polymerization temperature may be 0°C to 40°C, and the reaction time may be 10 minutes to 5 hours. In addition, the pH during the reaction may be maintained at 9 or more or 11 or more.

The solvent that can be used for the polymerization is not particularly limited as long as it is a solvent used for polymerization of polycarbonate in the art, and as an example, halogenated hydrocarbons such as methylene chloride and chlorobenzene can be used.

Moreover, the polymerization can be carried out in the presence of an acid binder. As the acid binder, an alkali metal hydroxide such as sodium hydroxide or potassium hydroxide, or an amine compound such as pyridine can be used.

Further, in order to adjust the molecular weight of the polycarbonate during the polymerization, polymerization can be performed in the presence of a molecular weight modifier. An alkylphenol having 1 to 20 carbon atoms may be used as the molecular weight modifier, and specific examples thereof include p-tert-butylphenol, p-cumylphenol, decylphenol, dodecylphenol, tetradecylphenol, hexadecylphenol, octadecylphenol, eicosylphenol, docosylphenol or triacontylphenol.

The molecular weight modifier can be added before, during or after the initiation of polymerization. The molecular weight modifier may be used in an amount of 0.01 to 10 parts by weight, or 0.1 to 6 parts by weight, based on 100 parts by weight of the aromatic diol compound, and a desired molecular weight can be obtained within this range.

In addition, in order to promote the polymerization reaction, a reaction accelerator such as a tertiary amine compound, a quaternary ammonium compound, or a quaternary phosphonium compound, including triethylamine, tetra-n-butylammonium bromide, or tetra-n-butylphosphonium bromide can be further used.

## 4. Molded Product

According to yet another embodiment of the invention, there can be provided a molded article comprising the recycled plastic of the other embodiment. The details of the recycled plastic includes all the contents described above in the other embodiments.

The molded article can be obtained by applying the recycled plastic to various known plastic molding methods without limitation. As an example of the molding method, injection molding, foam injection molding, blow molding, or extrusion molding may be mentioned.

Examples of the molded article are not particularly limited, and can be applied to various molded articles using plastic without limitation. Examples of the molded article include automobile parts, electrical and electronic products, communication products, daily necessities, building materials, optical components, exterior materials, and the like.

The molded article may further include one or more additives selected from the group consisting of an antioxidant, a plasticizer, an antistatic agent, a nucleating agent, a flame retardant, a lubricant, an impact enhancer, an optical brightener, an ultraviolet absorber, a pigment and a dye, if necessary, in addition to the recycled plastic of the other embodiments.

An example of the manufacturing method of the molded article may include a step of mixing the recycled plastic of the other embodiment and an additive well using a mixer, extrusion-molding the mixture with an extruder to produce pellets, drying the pellets, and then injecting them with an injection molding machine.

## [Advantageous Effects]

According to the present invention, a method for preparing a monomer composition for synthesizing recycled plastic that improves the efficiency of a recycling process while enabling an aromatic diol compound to have good yield and optical properties, with the aromatic diol compound being recovered through recycling by chemical decomposition of a polycarbonate-based resin, and a monomer composition for synthesizing recycled plastic, a recycled plastic and a molded product using the same can be provided.

## [DETAILED DESCRIPTION OF THE EMBODIMENTS]

Hereinafter, the present invention will be explained in detail with reference to the following examples. However, these examples are for illustrative purposes only, and the scope of the present invention is not limited thereto.

## <EXAMPLE and COMPARATIVE EXAMPLE: Preparation of recycled bisphenol A monomer composition>

Example 1

(1. Decomposition step) 14 mol of methylene chloride(MC), 6 mol of ethanol (EtOH) and 0.25 mol of sodium hydroxide (NaOH) were added to 3L high-pressure reactor, and the mixture was stirred. Then, 1.2 mol of waste polycarbonate (PC) was added thereto, and the mixture was stirred at 80°C to proceed a PC depolymerization reaction.

(2-1. First pH adjustment) The depolymerization reaction product (pH 14) containing bisphenol A was cooled to 30°C or less, and then a 10% HCl aqueous solution was added to the depolymerization reaction product so that the pH was adjusted to be 8, and the water layer was removed.

(2-2. Second pH adjustment) A 10% hydrochloric acid (HCl) aqueous solution was added to the depolymerization reaction product whose pH was adjusted to 8, so that the pH was adjusted to be 2, and the water layer was removed.

(3. Precipitation step) About 1500 g (about 83 mol) of water was added to the depolymerization reaction product obtained in the pH adjustment step, and the mixture was stirred to precipitate bisphenol A (BPA) crystals, and vacuum-filtered to recover bisphenol A (BPA).

(4. Purification step - Filtration) After that, bisphenol A was washed at 20~30°C using 300 g of methylene chloride (MC), and vacuum-filtered.

(5-1. Additional purification step - Redissolution step) After that, bisphenol A was added to 500 g of ethanol, and re-dissolved.

(5-2. Additional purification step - Adsorption step) After that, lignite activated carbon was added as an adsorbent at a ratio of 30% by weight relative to waste polycarbonate, purified through an adsorption tower, and then filtered to remove the lignite activated carbon.

(5-3. Additional purification step - Recrystallization step) After that, bisphenol A was recrystallized by adding 750 g of water, and then the resulting slurry was vacuum-filtered at 20~30°C to recover bisphenol A (BPA).

(6. Drying step) After that, the result was vacuum-dried in a convection oven at 40°C to prepare a recycled bisphenol A monomer composition in which recycled bisphenol A (BPA) has been recovered.

Example 2

A recycled bisphenol A monomer composition was prepared in the same manner as in Example 1, except that in (2-1. First pH adjustment step) of Example 1, the pH was adjusted to 9 instead of 8.

Comparative Example 1

(1. Decomposition step) 14 mol of methylene chloride(MC), 6 mol of ethanol(EtOH) and 0.25 mol of sodium hydroxide(NaOH) were added to 3L high-pressure reactor, and the mixture was stirred. Then, 1.2 mol of waste polycarbonate (PC) was added thereto, and the mixture was stirred at 80°C to proceed a PC depolymerization reaction.

(2. pH adjustment) The depolymerization reaction product (pH 14) containing bisphenol A was cooled to 30°C or less, and then a 10% HCl aqueous solution was added to the depolymerization reaction product so that the pH was adjusted to be 2, and the water layer was removed.

(3. Precipitation step) About 8000 g (about 444 mol) of water was added to the depolymerization reaction product obtained in the pH adjustment step, and the mixture was stirred to precipitate bisphenol A (BPA) crystals, and vacuum-filtered to recover bisphenol A (BPA).

(4. Purification step - Filtration) After that, bisphenol A was washed at 20~30°C using 300 g of methylene chloride (MC), and vacuum-filtered.

(5-1. Additional purification step-Redissolution step) After that, bisphenol A was added to 500 g of ethanol, and re-dissolved.

(5-2. Additional purification step - Adsorption step) After that, lignite activated carbon was added as an adsorbent at a ratio of 30% by weight relative to waste polycarbonate, purified through an adsorption tower, and then filtered to remove the lignite activated carbon.

(5-3. Additional purification step - Recrystallization step) After that, bisphenol A was recrystallized by adding 750 g of water, and then the resulting slurry was vacuum-filtered at 20 ~30°C to recover bisphenol A (BPA).

(6. Drying step) After that, the result was vacuum-dried in a convection oven at 40°C to prepare a recycled bisphenol A monomer composition in which recycled bisphenol A (BPA) has been recovered.

Comparative Example 2

A recycled bisphenol A monomer composition was prepared in the same manner as in Comparative Example 1, except that in (2. pH adjustment step) of Comparative Example 1, the pH was adjusted to 8 instead of 2.

**<Experimental Example>**

The physical properties were measured by the following methods, and the results are shown in Table 1 below.

**1. Purity**

After the recycled bisphenol A monomer composition was dissolved at 1 wt.% in an acetonitrile(ACN) solvent under the conditions of normal pressure and 20~30°C, and then the purity of the bisphenol A(BPA) was analyzed by UPLC (ultra performance liquid chromatography) via Waters HPLC system using ACQUITY UPLC®BEH C18 1.7 $\mu$m (2.1*50mm column).

**2. Color coordinates (L*, a*, b*)**

The color coordinate of the recycled bisphenol A monomer composition was analyzed in a reflection mode using a HunterLab UltraScan PRO Spectrophotometer.

### 3. Yield

The weight of BPA produced when the polycarbonate used in the reaction was 100% decomposed was measured, and the weight of the obtained BPA was measured, so that the yield of BPA was calculated as shown in the following Equation A.

$$[\text{Equation A}]$$

$$\text{Yield} (\%) = (W_1 / W_0) \times 100(\%)$$

wherein, in Equation A, $W_0$ is the mass of BPA obtained at the time of 100% decomposition, and $W_1$ is the mass of the actually obtained BPA. Specifically, when about 100 g of polycarbonate is decomposed, the mass of BPA obtained at the time of 100% decomposition in theory is 89 g. If the mass of the actually obtained BPA is 80 g, the yield is 80/89 * 100 = 90%.

### 4. Residual ethanol ratio

The depolymerization reaction product obtained in the pH adjustment step of the (3. Precipitation step) was diluted with methanol-d4 to a concentration of 10 mg/ml to prepare a sample, and the NMR spectrum detected through $^1$H NMR device was obtained, the area ratio of the ethanol peak among the ethanol peak and the bisphenol A (BPA) peak was measured, which was taken as the ratio of residual ethanol.

Specifically, ethanol was targeted at the peak at 3.63 ppm (quartet, 2H), and bisphenol A (BPA) was targeted at the peak at 6.67 ppm (4H), and the residual ethanol content was calculated through the following Equation B.

Residual ethanol ratio (%) = {(ethanol peak area in $^1$H NMR) / (ethanol peak area in $^1$H NMR + BPA peak area in $^1$H NMR)} X 100    [Equation B]

[Table 1]

| Measurement result of Experimental Example | | | | | | |
|---|---|---|---|---|---|---|
| Category | Purity (%) | L* | a* | b* | BPA yield (%) | Residual ethanol (%) |
| Example 1 | 99.5 | 98.03 | -0.12 | 0.59 | 98.8 | 0.5 |
| Example 2 | 99.4 | 97.6 | 0.06 | 0.75 | 98.6 | 0.7 |
| Comparative Example 1 | 97.2 | 95.67 | 0.16 | 1.72 | 21.1 | 1.6 |
| Comparative Example 2 | 98.9 | 97.4 | 0.06 | 1.47 | 18.2 | 2.2 |

As shown in Table 1, the recycled bisphenol A monomer compositions obtained in Examples 1 and 2 exhibited high purity of 99.4% to 99.5%. Also, the recycled bisphenol A monomer compositions obtained in Examples 1 and 2 exhibited color coordinates L* of 97.6 to 98.03, a* of -0.12 to 0.06, and b* of 0.59 to 0.75, showing excellent optical properties. Further, the recycled bisphenol A monomer compositions obtained in Examples 1 and 2 were measured to have a high BPA yield of 98.6% to 98.8%. Further, the recycled bisphenol A monomer composition obtained in Examples 1 and 2 was measured to have a low residual ethanol content of 0.5% to 0.7%.

On the other hand, the recycled bisphenol A monomer composition obtained in Comparative Examples 1 and 2 had a purity of 97.2% to 98.9%, which was lower than that of Examples. Also, the recycled bisphenol A monomer composition obtained in Comparative Examples 1 and 2 exhibited color coordinates L* of 95.67 to 97.4, a* of 0.06 to 0.16, and b* of 1.47 to 1.72, showing that the optical properties were inferior as compared to Examples.

In particular, the recycled bisphenol A monomer composition obtained in Comparative Examples 1 and 2 had a yield of 18.2% to 21.1% of the precipitated BPA, which was significantly lower than that of Examples, despite adding a much larger amount of water than in Examples. In addition, the recycled bisphenol A monomer composition obtained in Comparative Examples 1 and 2 was measured to have residual ethanol contents of 1.6% to 2.2%, which were higher than those of Examples.

**Claims**

1. A method for preparing a monomer composition for synthesizing recycled plastic, the method comprising the steps of:

   subjecting a polycarbonate-based resin to a depolymerization reaction;
   adjusting the pH of a depolymerization reaction product having a pH of 13 or more to be 8 to 12;
   adjusting the pH of the depolymerization reaction product whose pH is adjusted to 8 to 12 to be less than 4; and
   adding a crystallization solvent to the depolymerization reaction product whose pH is adjusted to less than 4 and recovering the formed aromatic diol compound crystals.

2. The method for preparing a monomer composition for synthesizing recycled plastic according to claim 1, wherein the pH difference value according to the following Equation 1 is 4 to 10:

   pH difference value = (pH of the depolymerization reaction product whose pH is adjusted to 8 to 12) - (pH of the depolymerization reaction product whose pH is adjusted to less than 4).　　　[Equation 1]

3. The method for preparing a monomer composition for synthesizing recycled plastic according to claim 1, wherein the pH difference value according to the following Equation 2 is 1 to 6:

   pH difference value = (pH of the depolymerization reaction product) - (pH of the depolymerization reaction product whose pH is adjusted to 8 to 12).　　　[Equation 2]

4. The method for preparing a monomer composition for synthesizing recycled plastic according to claim 1, wherein the adjusting the pH of the depolymerization reaction product to be 8 to 12 comprises:
   adding an acidic aqueous solution so that the pH of the depolymerization reaction product is 8 to 12.

5. The method for preparing a monomer composition for synthesizing recycled plastic according to claim 1, wherein:

   after the adjusting the pH of the depolymerization reaction product having a pH of 13 or more to be 8 to 12,
   the method further comprises removing a water layer among the layers divided into an organic solvent layer and the water layer.

6. The method for preparing a monomer composition for synthesizing recycled plastic according to claim 1, wherein:
   the adjusting the pH of the depolymerization reaction product whose pH is adjusted to 8 to 12 to be less than 4 comprises:
   adding an acidic aqueous solution so that the pH of the depolymerization reaction product whose pH is adjusted to 8 to 12 is less than 4.

7. The method for preparing a monomer composition for synthesizing recycled plastic according to claim 1, wherein:

   after the adjusting the pH of the depolymerization reaction product whose pH is adjusted to 8 to 12 to be less than 4,
   the method further comprises removing a water layer among the layers divided into an organic solvent layer and the water layer.

8. The method for preparing a monomer composition for synthesizing recycled plastic according to claim 1, wherein:
   the ratio of residual ethanol contained in the depolymerization reaction product whose pH is adjusted to less than 4 according to the following Equation 3 is less than 1%:

   --> Residual ethanol ratio (%) = {(ethanol peak area in $^{1}$H NMR) / (ethanol peak area in $^{1}$H NMR + aromatic diol compound peak area in $^{1}$H NMR)} X 100.　　　[Equation 3]

9. The method for preparing a monomer composition for synthesizing recycled plastic according to claim 1, wherein the depolymerization reaction of the polycarbonate-based resin proceeds in the presence of a solvent containing ethanol.

10. The method for preparing a monomer composition for synthesizing recycled plastic according to claim 9,wherein the amount of the ethanol is 1 mole to 5 moles relative to 1 mole of the polycarbonate-based resin.

11. The method for preparing a monomer composition for synthesizing recycled plastic according to claim 9, wherein the amount of the ethanol is 1 mole to 4 moles relative to 1 mole of the polycarbonate-based resin.

12. The method for preparing a monomer composition for synthesizing recycled plastic according to claim 1, wherein the depolymerization reaction of the polycarbonate-based resin proceeds by reacting a base in an amount of 0.5 mole or less relative to 1 mole of the polycarbonate-based resin.

13. The method for preparing a monomer composition for synthesizing recycled plastic according to claim 1, wherein: the adding the crystallization solvent to the depolymerization reaction product whose pH is adjusted to less than 4 and recovering the formed aromatic diol compound crystals comprises: adding 100 moles or less of water relative to 1 mole of the polycarbonate-based resin to the depolymerization reaction product whose pH is adjusted to less than 4 and precipitating aromatic diol compound crystals.

14. The method for preparing a monomer composition for synthesizing recycled plastic according to claim 1, wherein:

   after the adding a crystallization solvent to the depolymerization reaction product whose pH is adjusted to less than 4 and recovering the formed aromatic diol compound crystals,
   the method further comprises purifying aromatic diol compound.

15. The method for preparing a monomer composition for synthesizing recycled plastic according to claim 14, wherein : the purifying the aromatic diol compound comprises: washing the aromatic diol compound.

16. The method for preparing a monomer composition for synthesizing recycled plastic according to claim 14, wherein: the purifying the aromatic diol compound comprises: a dissolution step and an adsorption purification step of the aromatic diol compound.

17. The method for preparing a monomer composition for synthesizing recycled plastic according to claim 16, wherein:

   after the dissolution step and the adsorption purification step of the aromatic diol compound,
   the method further comprises recrystallizing the aromatic diol compound.

18. A monomer composition for synthesizing recycled plastic, comprising aromatic diol compound obtained by the method for preparing a monomer composition for synthesizing recycled plastic according to claim 1.

19. A recycled plastic comprising a reaction product of the monomer composition for synthesizing recycled plastic according to claim 18 and a comonomer.

20. A molded product comprising the recycled plastic according to claim 19.

## INTERNATIONAL SEARCH REPORT

| International application No. |
| --- |
| **PCT/KR2023/018958** |

### A. CLASSIFICATION OF SUBJECT MATTER

**C08J 11/10**(2006.01)i; **C08G 64/30**(2006.01)i; **C07C 37/055**(2006.01)i; **C07C 37/84**(2006.01)i; **C07C 39/16**(2006.01)i

According to International Patent Classification (IPC) or to both national classification and IPC

### B. FIELDS SEARCHED

Minimum documentation searched (classification system followed by classification symbols)

C08J 11/10(2006.01); C07C 37/055(2006.01); C08G 64/20(2006.01); C08G 64/30(2006.01); C08G 64/42(2006.01); C08J 11/16(2006.01); C08J 11/24(2006.01); G11B 7/26(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 폴리카보네이트계 수지(polycarbonate-based resin), 재활용 플라스틱 합성 (recycled plastic synthesis), 단량체(monomer), 비스페놀A(bisphenol A), 해중합(depolymerization), pH, 방향족 디올 화합물 (aromatic diol compound), 결정화 용매(crystallization solvent), 단계적 중화(stepwise neutralization)

### C. DOCUMENTS CONSIDERED TO BE RELEVANT

| Category* | Citation of document, with indication, where appropriate, of the relevant passages | Relevant to claim No. |
| --- | --- | --- |
| X | WO 2023-038266 A1 (LG CHEM, LTD.) 16 March 2023 (2023-03-16)<br>See paragraphs [0027], [0036]-[0045], [0097], [0104], [0156]-[0165], [0210] and [0211]; and claims 1, 3, 7, 17, 18 and 20. | 18-20 |
| A | | 1-17 |
| A | JP 2006-022183 A (TEIJIN CHEM LTD.) 26 January 2006 (2006-01-26)<br>See paragraphs [0009] and [0032]. | 1-20 |
| A | KR 10-2015-0028359 A (SAUDI BASIC INDUSTRIES CORPORATION) 13 March 2015 (2015-03-13)<br>See paragraphs [0073] and [0074]. | 1-20 |
| A | KR 10-2004-0055726 A (TEIJIN LIMITED) 26 June 2004 (2004-06-26)<br>See claims 1, 4, 7 and 10. | 1-20 |

☑ Further documents are listed in the continuation of Box C.　　☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **25 April 2024** | **26 April 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/018958** |

| C. | DOCUMENTS CONSIDERED TO BE RELEVANT | |
| --- | --- | --- |
| Category* | Citation of document, with indication, where appropriate, of the relevant passages | Relevant to claim No. |
| A | JP 2001-160243 A (VICTOR CO. OF JAPAN LTD.) 12 June 2001 (2001-06-12)<br>See claims 1 and 3. | 1-20 |

Form PCT/ISA/210 (second sheet) (July 2022)

**INTERNATIONAL SEARCH REPORT**
Information on patent family members

International application No.

**PCT/KR2023/018958**

| Patent document cited in search report | | | Publication date (day/month/year) | Patent family member(s) | | | Publication date (day/month/year) |
|---|---|---|---|---|---|---|---|
| WO | 2023-038266 | A1 | 16 March 2023 | CN | 116323773 | A | 23 June 2023 |
| | | | | CN | 116368179 | A | 30 June 2023 |
| | | | | CN | 116368180 | A | 30 June 2023 |
| | | | | CN | 116368181 | A | 30 June 2023 |
| | | | | CN | 116457403 | A | 18 July 2023 |
| | | | | CN | 116615492 | A | 18 August 2023 |
| | | | | EP | 4209528 | A1 | 12 July 2023 |
| | | | | EP | 4209529 | A1 | 12 July 2023 |
| | | | | EP | 4209530 | A1 | 12 July 2023 |
| | | | | EP | 4209531 | A1 | 12 July 2023 |
| | | | | EP | 4212572 | A1 | 19 July 2023 |
| | | | | EP | 4230679 | A1 | 23 August 2023 |
| | | | | JP | 2023-545936 | A | 01 November 2023 |
| | | | | JP | 2023-545937 | A | 01 November 2023 |
| | | | | JP | 2023-545938 | A | 01 November 2023 |
| | | | | JP | 2023-545953 | A | 01 November 2023 |
| | | | | JP | 2023-545954 | A | 01 November 2023 |
| | | | | JP | 2023-550948 | A | 06 December 2023 |
| | | | | KR | 10-2023-0039099 | A | 21 March 2023 |
| | | | | KR | 10-2023-0039100 | A | 21 March 2023 |
| | | | | KR | 10-2023-0039101 | A | 21 March 2023 |
| | | | | KR | 10-2023-0039102 | A | 21 March 2023 |
| | | | | KR | 10-2023-0045976 | A | 05 April 2023 |
| | | | | KR | 10-2023-0052755 | A | 20 April 2023 |
| | | | | TW | 202311212 | A | 16 March 2023 |
| | | | | TW | 202311213 | A | 16 March 2023 |
| | | | | TW | 202311396 | A | 16 March 2023 |
| | | | | TW | 202311397 | A | 16 March 2023 |
| | | | | TW | 202313543 | A | 01 April 2023 |
| | | | | TW | 202315855 | A | 16 April 2023 |
| | | | | TW | I812353 | B | 11 August 2023 |
| | | | | US | 2023-0374251 | A1 | 23 November 2023 |
| | | | | US | 2023-0382837 | A1 | 30 November 2023 |
| | | | | US | 2023-0383089 | A1 | 30 November 2023 |
| | | | | US | 2023-0383090 | A1 | 30 November 2023 |
| | | | | US | 2023-0391702 | A1 | 07 December 2023 |
| | | | | US | 2024-0002627 | A1 | 04 January 2024 |
| | | | | WO | 2023-038267 | A1 | 16 March 2023 |
| | | | | WO | 2023-038268 | A1 | 16 March 2023 |
| | | | | WO | 2023-038269 | A1 | 16 March 2023 |
| | | | | WO | 2023-038270 | A1 | 16 March 2023 |
| | | | | WO | 2023-038271 | A1 | 16 March 2023 |
| JP | 2006-022183 | A | 26 January 2006 | JP | 4272123 | B2 | 03 June 2009 |
| KR | 10-2015-0028359 | A | 13 March 2015 | CN | 104718238 | A | 17 June 2015 |
| | | | | CN | 104718238 | B | 24 August 2016 |
| | | | | DE | 112013006107 | T5 | 17 September 2015 |
| | | | | JP | 2015-533816 | A | 26 November 2015 |
| | | | | JP | 5881909 | B2 | 09 March 2016 |
| | | | | KR | 10-1565917 | B1 | 05 November 2015 |
| | | | | US | 8680227 | B1 | 25 March 2014 |

Form PCT/ISA/210 (patent family annex) (July 2022)

**INTERNATIONAL SEARCH REPORT**
Information on patent family members

International application No.

**PCT/KR2023/018958**

| Patent document cited in search report | | | Publication date (day/month/year) | Patent family member(s) | | | Publication date (day/month/year) |
|---|---|---|---|---|---|---|---|
| | | | | WO | 2014-099596 | A1 | 26 June 2014 |
| KR | 10-2004-0055726 | A | 26 June 2004 | CN | 1481347 | A | 10 March 2004 |
| | | | | CN | 1481347 | C | 10 March 2004 |
| | | | | EP | 1439158 | A1 | 21 July 2004 |
| | | | | TW | 229100 | A | 11 March 2005 |
| | | | | TW | 229100 | B | 11 March 2005 |
| | | | | TW | I229100 | B | 11 March 2005 |
| | | | | US | 2004-0054238 | A1 | 18 March 2004 |
| | | | | WO | 03-035592 | A1 | 01 May 2003 |
| | | | | WO | 2003-035592 | A1 | 10 February 2005 |
| JP | 2001-160243 | A | 12 June 2001 | None | | | |

Form PCT/ISA/210 (patent family annex) (July 2022)

**EP 4 524 187 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230098843